(19) Europäisches Patentamt
European Patent Office
Office européen des brevets

(11) EP 4 613 191 A1

(12) EUROPEAN PATENT APPLICATION
published in accordance with Art. 153(4) EPC

(43) Date of publication:
**10.09.2025 Bulletin 2025/37**

(21) Application number: **23885828.6**

(22) Date of filing: **01.11.2023**

(51) International Patent Classification (IPC):
***A61B 5/11** (2006.01)*

(52) Cooperative Patent Classification (CPC):
**A61B 5/11**

(86) International application number:
**PCT/JP2023/039485**

(87) International publication number:
**WO 2024/096074 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 JP 2022177386**

(71) Applicant: **MEIJI CO., LTD**
**Chuo-ku**
**Tokyo 104-8306 (JP)**

(72) Inventors:
- **KUBOTA, Takayuki**
**Hachioji-shi, Tokyo 192-0919 (JP)**
- **MATSUO, Atsushi**
**Hachioji-shi, Tokyo 192-0919 (JP)**

(74) Representative: **TBK**
**Bavariaring 4-6**
**80336 München (DE)**

(54) **CHEWING MOTION ANALYSIS METHOD**

(57) A masticatory movement analysis method includes, an acquisition step of measuring myoelectric potential from muscles at a plurality of measurement spots on a face when a target person chews and acquiring myoelectric potential data, a calculation step of calculating a plurality of feature amounts from unit data obtained by dividing the myoelectric potential data on a basis of a unit time, and a determination step of determining from the feature amounts which one of tongue movement, cheek movement, and occlusal movement is being performed by the target person during acquisition of the unit data.

Fig. 3

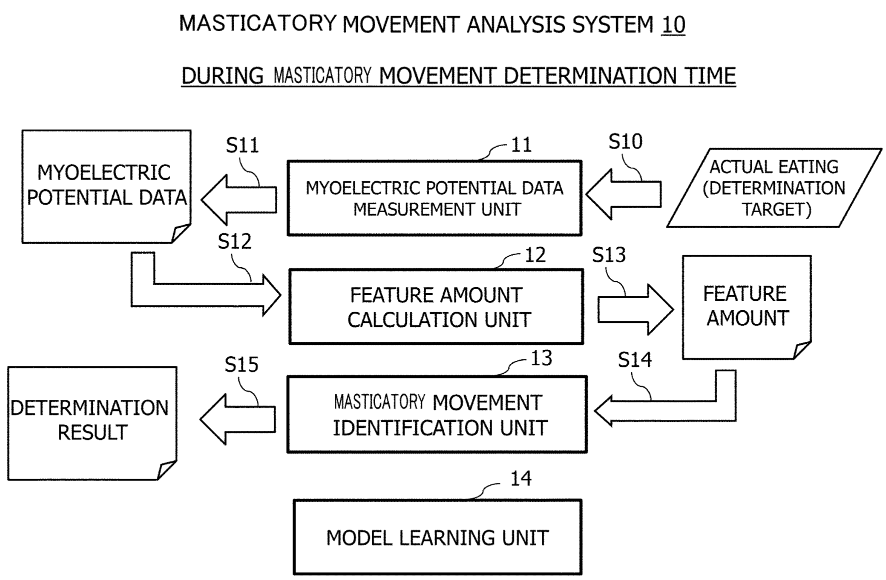




Processed by Luminess, 75001 PARIS (FR)

## Description

### TECHNICAL FIELD

The present invention relates to a masticatory movement analysis method.

### BACKGROUND ART

In recent years, research on mastication has garnered attention. Mastication is, for example, a series of processes in which a person finely crushes food to form a paste-like bolus that can be swallowed. The effects of mastication have garnered, for example, attention for their relevance to various factors in life activities, such as efficient absorption of nutrients contained in boluses, maintaining healthy oral environments, influencing mental conditions or brain functions, and preventing diseases.

As a method for evaluating mastication, there has been, for example, a method in which the number of chewing times, chewing speed, occlusal strength, or the like is measured and evaluated. According to this method, the degree to which the movement of chewing foods is promoted can be compared by, for example, changing target chewing foods.

On the other hand, this method evaluates only chewing movements and cannot provide satisfactory evaluation of masticatory functions. Masticatory movements include not only simply chewing movements but also various movements. By comprehensively evaluating these movements, it is possible to grasp the detailed nature of masticatory movements and specifically evaluate how masticatory movements vary depending on person's mastication performance or differences in food characteristics.

Technologies related to the evaluation of mastication have been disclosed in the following Patent Literatures.

### CITATION LIST

### PATENT LITERATURE

Patent Literature 1: Japanese Patent Application Laid-open No. 2012-232065
Patent Literature 2: WO 2019/022259

### SUMMARY OF INVENTION

### PROBLEMS TO BE SOLVED BY THE INVENTION

However, a method for comprehensively evaluating masticatory movements has not been established. In the evaluation of masticatory movements, it has not been known which movement, other than chewing, is measured and analyzed to enable appropriate evaluation of masticatory movements.

Therefore, one disclosure provides a masticatory movement analysis method that enables comprehensive evaluation of masticatory movements.

### MEANS FOR SOLVING THE PROBLEMS

The present invention provides, for example, the following means.

(1) A mastication movement analysis method includes, an acquisition step of measuring myoelectric potential from muscles at a plurality of measurement spots on a face when a target person chews and acquiring myoelectric potential data; a calculation step of calculating a plurality of feature amounts from unit data obtained by dividing the myoelectric potential data on a basis of a unit time; and a determination step of determining from the feature amounts which one of tongue movement, cheek movement, and occlusal movement is being performed by the target person during acquisition of the unit data.

(2) The masticatory movement analysis method according to (1), wherein the plurality of measurement spots include a right masseter muscle, a left masseter muscle, and a muscle near a central portion of a suprahyoid muscle group.

(3) The masticatory movement analysis method according to (1) or (2), wherein the determination step includes determining whether a tongue is being moved upward, downward, rightward, or leftward, whether the tongue is being moved forward or backward, and whether the tongue is wiping an inner or outer side of a right or left tooth in the tongue movement, and determining whether a molar is being wiped by a left cheek, a right cheek, or both the left and right cheeks in the cheek movement.

(4) The masticatory movement analysis method according to any one of (1) to (3), wherein the feature amounts are calculated from a waveform of myoelectric potential for each of the measurement spots and from a waveform data group obtained by aggregating myoelectric potential from the plurality of measurement spots.

(5) The masticatory movement analysis method according to any one of (1) to (4), wherein first unit data partially overlaps with second unit data, the second unit data being data from a time prior to the first unit data.

(6) The masticatory movement analysis method according to any one of (1) to (5), wherein the determination step includes calculating a probability of each movement being performed in the determination as to which one of the movements is being performed.

(7) The masticatory movement analysis method according to any one of (1) to (6), wherein the determination step is performed by a learned model that has performed learning using myoelectric potential data from the plurality of measurement spots obtained when the tongue movement, the cheek movement, and the occlusal movement are performed as training data.

(8) The masticatory movement analysis method according to any one of (1) to (7), wherein the training data includes myoelectric potential data from the plurality of measurement spots during a rest time at which mastication is not performed.

(9) The masticatory movement analysis method according to any one of (1) to (8), wherein the training data includes myoelectric potential data from the plurality of measurement spots during a swallowing time at which swallowing movement is performed.

(10) The masticatory movement analysis method according to any one of (1) to (9), wherein the determination step includes determining that movement during the rest time and movement during the swallowing time do not correspond to any of the tongue movement, the cheek movement, and the occlusal movement.

(11) The masticatory movement analysis method according to any one of (1) to (10), wherein the plurality of measurement spots further include left and right sides of the suprahyoid muscle group.

(12) The masticatory movement analysis method according to any one of (1) to (11), wherein the plurality of measurement spots further include left and right temporal muscles.

(13) The masticatory movement analysis method according to any one of (1) to (12), wherein the plurality of measurement spots further include left and right cheek muscles.

(14) The masticatory movement analysis method according to any one of (1) to (13), including: a food difference evaluation step of detecting a difference in masticatory movement resulting from a different food type, on a basis of a determination result obtained when the target person chews a different food.

(15) The masticatory movement analysis method according to any one of (1) to (14), including: an elapsed time evaluation step of detecting a change in masticatory movement over time.

ADVANTAGEOUS EFFECTS OF THE INVENTION

One disclosure enables comprehensive evaluation of masticatory movements.

BRIEF DESCRIPTION OF DRAWINGS

FIG. 1 is a diagram illustrating a configuration example of a masticatory movement analysis system 10.

FIG. 2 is a diagram illustrating a configuration example of the masticatory movement analysis device 100.

FIG. 3 is a diagram illustrating the software configuration of the masticatory movement analysis system 10 and an example of the input/output data of each unit during the masticatory movement determination time.

FIG. 4 is a diagram illustrating the software configuration of the masticatory movement analysis system 10 and an example of the input/output data of each unit during the model learning time.

FIG. 5 is a diagram illustrating an example of myoelectric potential data from each measurement spot.

FIG. 6 is a diagram illustrating an example of a processing flowchart for the feature amount calculation processing S100.

FIG. 7 is a diagram illustrating an example of the myoelectric potential data and the unit data.

FIG. 8 is a diagram illustrating an example of the calculated feature amounts.

FIG. 9 is a diagram illustrating an example of a processing flowchart for the masticatory movement analysis processing S200.

FIG. 10 is a diagram illustrating an example of determined masticatory movements.

FIGS. 11A and 11B are diagrams illustrating an example of masticatory movement determination results.

FIG. 12 is a diagram illustrating an example of a processing flowchart for the model learning processing S300.

FIG. 13 is a diagram illustrating an example of masticatory movements based on differences in chewing objects.
FIG. 14 is a diagram illustrating an example of changes in masticatory movements based on the elapse of chewing time.
FIG. 15 is a diagram illustrating an example of real-time display of masticatory movement analysis.
FIG. 16 is a diagram illustrating an example of masticatory movements including swallowing based on differences in chewing objects.
FIG. 17 is a diagram illustrating an example of changes in masticatory movements including swallowing.

DESCRIPTION OF EMBODIMENTS

[First Embodiment]

A first embodiment will be described.

<Configuration Example of Masticatory Movement Analysis System 10>

FIG. 1 is a diagram illustrating a configuration example of a masticatory movement analysis system 10. The masticatory movement analysis system 10 has a masticatory movement analysis device 100 and myoelectric potential measurement pads P1 to P3. The masticatory movement analysis system 10 is a system that measures myoelectric potential from the facial muscles of a measurement target person 20 and determines and analyzes which masticatory movement the measurement target person 20 has performed on the basis of the measurement data.

The myoelectric potential measurement pads P1 to P3 are pads that are placed on the face of the measurement target person 20 performing masticatory movements and measure myoelectric potential indicating the movement amounts of the facial muscles of the measurement target person 20. The myoelectric potential measurement pads P1 to P3 transmit the measured myoelectric potential, for example, to the masticatory movement analysis device 100 via cables. Note that the three myoelectric potential measurement pads are provided in FIG. 1, but this number can be increased according to measurement spots.

The masticatory movement analysis device 100 is a device that acquires measured myoelectric potential data and determines and analyzes which masticatory movement the measurement target person 20 has performed on the basis of the measured data, and is, for example, a computer. The masticatory movement analysis device 100 outputs the movement determination results as data or displays the same on a screen.

<Configuration Example of Masticatory Movement Analysis Device 100>

FIG. 2 is a diagram illustrating a configuration example of the masticatory movement analysis device 100. The masticatory movement analysis device 100 has a CPU (Central Processing Unit) 110, a storage 120, a memory 130, a communication circuit 140, and a display 150.

The storage 120 is an auxiliary storage device such as a flash memory, an HDD (Hard Disk Drive), and an SSD (Solid State Drive) that stores programs or data. The storage 120 stores a myoelectric potential measurement program 121, a feature amount calculation program 122, a masticatory movement identification model 123, and a model learning program 124.

The masticatory movement identification model 123 is, for example, an AI (artificial intelligence), and can determine which masticatory movement is being performed (masticatory movement) on the basis of the feature amounts of measured myoelectric potential data and output the probability of each masticatory movement being performed. The masticatory movement identification model 123 uses a learned model when determining masticatory movement. The masticatory movement identification model 123 improves determination accuracy for the masticatory movement along with the input of training data for learning. In the following description, unless otherwise particularly mentioned, the masticatory movement identification model 123 is a learned model that has completed learning by a prescribed amount (prescribed number of times) and achieves determination accuracy at a prescribed level or higher.

The memory 130 is a region into which the programs stored in the storage 120 are loaded. Further, the memory 130 may also be used as a region in which the programs store data.

The communication circuit 140 is a device that receives signals detected by the myoelectric potential measurement pads P1 to P3 through (a receiver included in) a myoelectric potential data measurement unit 11 and acquires myoelectric potential data via the cables.

The display 150 is a screen or a display unit that shows movement determination results or analysis results. The display 150 may be, for example, an integrated type or a type connected to the outside.

The CPU 110 is a processor that loads the programs stored in the storage 120 into the memory 130, runs the loaded programs, constructs each unit, and realizes each processing.

The CPU 110 constructs the myoelectric potential data measurement unit and performs myoelectric potential data measurement processing by running the myoelectric potential measurement program 121. The myoelectric potential data measurement processing is processing for causing the myoelectric potential measurement pads P1 to P3 to measure myoelectric potential and transmit myoelectric potential data to acquire the same.

The CPU 110 constructs a feature amount calculation unit and performs feature amount calculation processing by running the feature amount calculation program 122. The feature amount calculation unit is processing for segmenting input myoelectric potential data on the basis of a determination unit (time unit) and calculating feature amounts for each segmented sectional data.

The CPU 110 constructs a masticatory movement identification unit and performs masticatory movement identification processing by performing the masticatory movement identification model 123. The masticatory movement identification processing is processing for outputting masticatory movements corresponding to input feature amounts. The masticatory movement identification processing includes, for example, calculating the probability of each masticatory movement being performed and outputting the calculated probability.

The CPU 110 constructs a model learning unit and performs model learning processing by running the model learning program 124. The model learning processing is processing for generating training data that is input when the masticatory movement identification model 123 (including a learned model or an unlearned model) is caused to perform learning. The training data is made up of, for example, the feature amounts of myoelectric potential data obtained by measuring intentional masticatory movements and the types (answer data) of the intentional movements. The model learning processing is processing for generating or editing the training data and inputting the same to the masticatory movement identification model 123 for learning.

<Software Configuration Example of Masticatory Movement Analysis System 10>

The masticatory movement analysis system 10 has the myoelectric potential data measurement unit 11, a feature amount calculation unit 12, a masticatory movement identification unit 13, and a model learning unit 14. Hereinafter, the processing and input/output of each unit during the masticatory movement determination time when masticatory movements are determined and the model learning time when the masticatory movement identification model 123 performs learning will be described.

<1. During Masticatory Movement Determination Time>

FIG. 3 is a diagram illustrating the software configuration of the masticatory movement analysis system 10 and an example of the input/output data of each unit during the masticatory movement determination time.

The myoelectric potential data measurement unit 11 measures myoelectric potential data in actual eating (mastication of food) performed by a determination target person (S10), and outputs the measured myoelectric potential data (S11). The myoelectric potential data measurement unit 11 measures the myoelectric potential data for a prescribed time using, for example, the myoelectric potential measurement pads P1 to P3.

The feature amount calculation unit 12 receives the myoelectric potential data (S12) and outputs feature amounts (S13). The feature amount calculation unit 12 segments the myoelectric potential data on the basis of a time unit and calculates feature amounts for each segmented unit data. Further, the feature amount calculation unit 12 may also calculate feature amounts in consideration of unit data from a plurality of measurement spots. Moreover, when calculating feature amounts on the basis of unit data from one measurement spot, the feature amount calculation unit 12 may also consider unit data at other times.

The masticatory movement identification unit 13 receives the feature amounts (S14), and outputs the probability of masticatory movement for each unit data (probability indicating which masticatory movement was performed during the acquisition time of the unit data) as a determination result (S15).

The model learning unit 14 is a processing unit that operates during the learning time of the masticatory movement identification model 123, and does not particularly operate during the masticatory movement determination time.

<2. During Model Learning Time>

FIG. 4 is a diagram illustrating the software configuration of the masticatory movement analysis system 10 and an example of the input/output data of each unit during the model learning time.

The myoelectric potential data measurement unit 11 measures myoelectric potential data (S20) and outputs the measured myoelectric potential data (S21) during simulated movement for generating training data. The simulated movement is, for example, movement extracted from masticatory movement, movement using the tongue, movement using the oral inner walls of the cheeks, or the like in actual eating. In this case, the myoelectric potential data measurement unit 11 measures myoelectric potential data during actual eating.

Further, the simulated movement may also be movement that is intentionally performed by a subject, such as masticatory movement for learning, masticatory movement for improving determination accuracy, and masticatory movement that is less likely to be performed during normal mastication.

The feature amount calculation unit 12 receives the myoelectric potential data (for training data) (S22) and outputs feature amounts (for training data) (S23).

The model learning unit 14 receives the feature amounts (for training data) (S24), edits the feature amount data, generates answer data indicating which masticatory movement was performed during the simulated movement, and outputs the edited feature amount data and the answer data as training data (S25).

The masticatory movement identification unit 13 receives the training data (S26) and performs the learning of the masticatory movement identification model 123.

<Masticatory Movement Determination Processing>

Each processing of masticatory movement determination processing for determining masticatory movement will be described.

<1. Myoelectric Potential Data Measurement Processing>

Myoelectric potential data measurement processing is processing for measuring myoelectric potential from each facial muscle of a measurement target person, and is performed by, for example, the myoelectric potential data measurement unit 11. The myoelectric potential measurement processing is, for example, processing for placing the myoelectric potential measurement pads P1 to P3 on the face of the measurement target person and causing the measurement target person to chew to measure myoelectric potential.

The muscles of the face that are measurement targets are, for example, a masseter muscle on the right side (that may be called the right masseter muscle below), a masseter muscle on the left side (that may be called the left masseter muscle below), and a muscle near the central portion of a suprahyoid muscle group (that may be called the central portion of the suprahyoid muscle group below).

FIG. 5 is a diagram illustrating an example of myoelectric potential data from each measurement spot. FIG. 5 illustrates three graphs. From top to bottom, the graphs sequentially illustrate measurement values at the right masseter muscle, the left masseter muscle, and the central portion of the suprahyoid muscle group. The graphs illustrate measured myoelectric potential on the vertical axis and measurement time on the horizontal axis.

When the myoelectric potential is, for example, 0, it indicates that the muscles are not active. The greater an absolute value, the greater the amounts of active muscle fibers. For example, in FIG. 5, the times when the myoelectric potential significantly increases in the vertical direction indicate that the muscle fibers at the measurement spots are highly active.

<2. Feature Amount Calculation Processing>

Feature amount calculation processing S100 is processing for calculating feature amounts from myoelectric potential data, and is performed by, for example, the feature amount calculation unit 12. FIG. 6 is a diagram illustrating an example of a processing flowchart for the feature amount calculation processing S100.

The feature amount calculation unit 12 receives myoelectric potential data (S100-1).

The feature amount calculation unit 12 segments the myoelectric potential data on the basis of a time unit for each prescribed time to generate unit data (S100-2).

FIG. 7 is a diagram illustrating an example of the myoelectric potential data and the unit data. The upper graph in FIG. 7 is a graph where the measurement data from each portion in FIG. 5 overlap with each other.

The feature amount calculation unit 12 segments the myoelectric potential data on the basis of a prescribed time unit T1 to generate unit data D1. Next, the feature amount calculation unit 12 uses the time elapsed by a shift amount T2 after the start time of the unit data D1 as a start time, and segments the myoelectric potential data on the basis of the time unit T1 to generate unit data D2. The feature amount calculation unit 12 repeatedly shifts a start time by the shift amount T2 and segments the myoelectric potential data on the basis of the prescribed time unit T1 to generate unit data Dn (where n is an integer).

In the feature amount calculation processing S100, the waveform of myoelectric potential data can be fully used to determine masticatory movements by setting a shift amount and overlapping the times of unit data with each other. Further, in the feature amount calculation processing S100, feature amounts considering the trend (change trend) of the waveform of the myoelectric potential data can be calculated by partially overlapping the times of the unit data with each other.

Note that the feature amount calculation unit 12 generates unit data for each measurement spot. In this case, the feature amount calculation unit 12 calculates feature amounts for each measurement spot. Further, the feature amount calculation unit 12 may also generate unit data from a waveform data group obtained by aggregating the unit data and a synthetic wave obtained by synthesizing the myoelectric potential data from a plurality of measurement spots. In this case, the feature amount calculation unit 12 calculates feature amounts reflecting the measurement results from the plurality of measurement spots. Moreover, the feature amount calculation unit 12 may also generate unit data from any of a waveform data group obtained by aggregating the unit data for each measurement spot and a synthetic wave. In this case, the feature amount calculation unit 12 calculates both feature amounts for each measurement spot and feature amounts reflecting measurement results from a plurality of measurement spots. In this case, masticatory movements are determined on the basis of feature amounts considering both the trend of individual measurement spots and the overall trend of the facial muscles. Compared to determining masticatory movements on the basis of feature amounts that consider only individual measurement spots, waveform data obtained by aggregating unit data, or synthesis, this method may achieve more accurate determinations.

Referring back to FIG. 6, the feature amount calculation unit 12 calculates feature amounts for each unit data (S100-3) and ends the processing.

FIG. 8 is a diagram illustrating an example of the calculated feature amounts. The calculated feature amounts include, for example, a root mean square, a mean absolute value, an integral value, a zero crossing rate, the number of extreme values, a wavelength, a median frequency, a mean frequency, an n-th order coefficient of an autoregression model (where n is an integer of 1 to 6), and the like.

The root mean square is, for example, the square root of the arithmetic mean of squared measurement values. The root mean square indicates, for example, a feature amount related to the amplitude of the waveform of measurement data.

The mean absolute value is, for example, the average of the absolute values of measurement values. The mean absolute value indicates, for example, a feature amount related to the amplitude of the waveform of measurement data.

The integral value is, for example, a sectional area calculated by multiplying the measurement values between unit data by a sampling time for myoelectric potential and summing all the products. The integral value indicates, for example, a feature amount related to the amplitude of the waveform of measurement data.

The zero crossing rate is, for example, the number of times where measurement data changes from a number other than 0 to 0 (counting 1 time when 0 continues). The zero crossing rate indicates, for example, a feature amount related to the frequency of the waveform of measurement data.

The number of extreme values is, for example, the number of times (peaks of mountains) where measurement data reaches its highest point in a series of times. The number of extreme values indicates, for example, a feature amount related to the frequency of the waveform of measurement data.

The wavelength is, for example, the wavelength of the waveform of measurement data. The wavelength indicates, for example, a feature amount related to the frequency of the waveform of measurement data.

The median frequency is, for example, the frequency at which the power spectrum of frequencies obtained through fast Fourier transformation of measurement data is divided into two equal areas. The median frequency indicates, for example, a feature amount related to the frequency of the waveform of measurement data.

The mean frequency is, for example, the average value of each frequency in the power spectrum of frequencies obtained through fast Fourier transformation of measurement data. The mean frequency indicates, for example, a feature amount related to the frequency of the waveform of measurement data.

The autoregression model is, for example, a model that regresses an output at a certain time using an output at an earlier time. The autoregression model indicates, for example, a feature amount related to the trend of the waveform of measurement data. The autoregression model is represented by, for example, the following expression (1).

$$X_t = a_1X_{t-1} + a_2X_{t-2} + \ldots + a_nX_{t-n} \ldots \text{Expression (1)}$$

$X_t$ represents an output at time t, and $a_n$ represents a coefficient related to an output at time n, which is called an n-th-order coefficient. The feature amount calculation unit 12 calculates, for example, the first to sixth order coefficients as feature amounts.

In the masticatory movement analysis system 10, the feature amount calculation unit 12 calculates many feature amounts as described above and inputs the calculated feature amounts to the masticatory movement identification unit 13, enabling more accurate analysis of masticatory movements.

<3. Masticatory Movement Analysis Processing>

Masticatory movement analysis processing S200 is processing for calculating feature amounts from myoelectric potential data, and is performed by, for example, the masticatory movement identification unit 13. FIG. 9 is a diagram illustrating an example of a processing flowchart for the masticatory movement analysis processing S200.

The masticatory movement identification unit 13 receives feature amounts (S200-1).

On the basis of the received feature amounts, the masticatory movement identification unit 13 analyzes the feature amounts using a masticatory movement analysis method (S200-2).

The masticatory movement analysis method will be described.

The masticatory movement analysis method is, for example, an analysis method for estimating masticatory movements using a decision tree constructed by learning of the masticatory movement identification model 123. The generation of the decision tree will be described in the section of model learning processing.

The masticatory movement identification unit 13 branches each feature amount and calculates movements (or the probabilities of the movements) corresponding to the feature amounts using the decision tree.

The masticatory movement identification unit 13 calculates the probability of each masticatory movement being performed (S200-3) and ends the processing.

FIG. 10 is a diagram illustrating an example of determined masticatory movements. The masticatory movement identification unit 13 calculates the probability of each movement being performed, for example, for 15 different movements.

The determined movements are classified into four movements as major categories. The first movement refers to occlusal movements and, for example, indicates how the teeth move vertically (how the teeth chew). The occlusal movements include, for example, masticatory movements.

The second movement refers to cheek movements and, for example, indicates how the cheeks move. The cheek movements include, for example, movements such as wiping the molars with both cheeks, wiping the molars with the right cheek, and wiping the molars with the left cheek.

The third movement refers to tongue movements and, for example, indicates how the tongue moves. The tongue movements include, for example, movements such as wiping the right teeth (the inner side or occlusal surface), wiping the right teeth (the outer side), wiping the left teeth (the inner side or occlusal surface), and wiping the left teeth (the outer side) upward, downward, forward, backward, rightward, and leftward.

The fourth movement refers to other movements and, for example, indicates a stationary state. Other movements include, for example, movements such as being at rest.

FIG. 11 is a diagram illustrating an example of masticatory movement determination results. FIG. 11 illustrates the probabilities of masticatory movements being performed.

FIG. 11(A) is a diagram illustrating an example of a graph indicating the probability of masticatory movement being performed before smoothing. The graph indicates the probability of masticatory movement being performed on the vertical axis and time (unit data) on the horizontal axis.

FIG. 11(B) is a diagram illustrating an example of a graph indicating the probability of masticatory movement being performed after smoothing. The smoothing is performed, for example, by generating the average movement data of a plurality of (for example, three) unit data instead of using one unit data. It appears from FIG. 11(B) that a waveform change indicating that the probability of masticatory movement being performed is high is smoother compared to FIG. 11(A). In actual mastication, for example, abrupt movement changes such as suddenly stopping masticatory movement and suddenly starting masticatory movement do not occur frequently, and movements gradually transition from one to another in many cases. Therefore, by using smoothed data as described above, a change in the probabilities of calculated masticatory movements is smoothed during a series of mastication, enabling more accurate estimation of the masticatory movements.

<4. Model Learning Processing>

Model learning processing S300 is processing for causing the masticatory movement identification model 123 to perform learning, and is performed by, for example, the model learning unit 14. FIG. 12 is a diagram illustrating an example of a processing flowchart for the model learning processing S300.

The model learning unit 14 causes, for example, the myoelectric potential data measurement unit 11 to measure myoelectric potential data during simulated movement (S300-1).

Next, the model learning unit 14 causes, for example, the feature amount calculation unit 12 to perform feature amount calculation processing and output the feature amounts of the myoelectric potential data (S300-2).

Next, the model learning unit 14 combines the feature amounts and answer data (data indicating which masticatory movement was performed during the simulated movement) together to generate training data (S300-3).

Then, the model learning unit 14 inputs the training data to the masticatory movement identification model 123 to perform learning (S300-4), and ends the processing.

The masticatory movement identification model 123 recognizes the relationship (trend) between the feature amounts and the masticatory movement performed when the feature amounts are indicated, on the basis of training data to perform learning.

There are relationships between myoelectric potential data and masticatory movements. For example, in masticatory movements, the activity amounts of the left and right masseter muscles are often extremely greater than those of the suprahyoid muscle group. On the other hand, in tongue movements such as moving the tongue upward and wiping the right outer side with the tongue, the activity of the suprahyoid muscle group is often extremely greater than that of the left and right masseter muscles. Further, in cheek-using movements such as wiping with both cheeks, the difference in activity between the left and right masseter muscles and the suprahyoid muscle group is often smaller compared to the masticatory movements or the tongue movements described above.

The masticatory movement identification model 123 learns, for example, the trend of the relationships between myoelectric potential data and masticatory movements as described above from feature amounts, and estimates the masticatory movements.

In performing learning, the masticatory movement identification model 123 generates, for example, the branches of a decision tree used to estimate masticatory movements from feature amounts, or generates thresholds for the branches. If learning has been already completed, the masticatory movement identification model 123, for example, changes (including adding or deleting) the branches or changes (refines) the thresholds for the branches to increase the accuracy of estimation of masticatory movements.

Further, the masticatory movement identification model 123 may be other than a decision tree, such as a neural network and other AI. That is, the masticatory movement identification model 123 only needs to be an AI that can learn the relationships between myoelectric potential data obtained by measuring each portion of the face and masticatory movements as training data, and estimate the masticatory movements on the basis of the myoelectric potential data from each portion of the face.

In the model learning processing S300, for example, all the 15 masticatory movements are performed as simulated movements to measure myoelectric potential. Further, when performing the simulated movements, movement speeds or rhythms may be changed to measure myoelectric potential.

In the model learning processing S300, in order to calculate feature amounts during transition from one masticatory movement to another, two or more different masticatory movements may be combined and sequentially performed to measure myoelectric potential. Moreover, in the model learning processing S300, for example, calculated feature amounts may undergo mathematical processing, or trends in the feature amounts may be emphasized to generate new feature amounts as training data.

Further, in the model learning processing S300, actually-determined measurement data other than simulated movements may also be used as training data. In this case, it is necessary to separately record the relationships between masticatory movements and times (indicating when the masticatory movements were performed) during measurement.

Note that measurement data from each measurement spot at rest (where mastication is not being performed) may also be used as training data. Even when masticatory is not being performed, a target person moves muscles to a small extent, for example, during breathing, blinking, or the like. Therefore, by using measurement data at rest as training data, it is possible to prevent, for example, myoelectric potential data indicating small activity amounts at rest from being falsely recognized as masticatory movements. Further, by using measurement at rest as training data, it is possible to remove, for example, measurement data at rest as stationary noise data, resulting in an improvement in the accuracy of determination of masticatory movements.

<Evaluations Using Determination Results of Masticatory Movements>

Results obtained by determining masticatory movements can be used in various evaluations. Hereinafter, the evaluations will be described.

<1. Evaluations of Masticatory Movements Based on Differences in Chewing Objects>

FIG. 13 is a diagram illustrating an example of masticatory movements based on differences in chewing objects. FIG. 13 illustrates the masticatory movements and their proportions (percentages) for foods A and B. Note that in FIG. 13, the masticatory movements are selected as those performed with the highest probability on the basis of unit data.

According to FIG. 13, cheek movements during chewing are greater in the food B than in the food A. For example, if it is assumed that cheek movements promote the activity of facial muscles, the food B could be advertised and sold as "food for preventing cheek sagging."

Further, according to FIG. 13, movements for moving the tongue to the outer side of the teeth are greater in the food A than in the food B. For example, if it is assumed that the large movements of the tongue to the outer side increase the activity amount of the tongue and promote the production of saliva, the food A could be advertised and sold as "food for increasing the production amount of saliva."

By detecting and evaluating differences in masticatory movements for each food as described above, it is possible to recognize which movements of portions (such as the tongue, teeth, and cheeks) the food promotes and advertise and sell the food as food based on various effects.

Further, for example, in medical rehabilitation or the like, it is possible to propose or offer foods for estimating the activity level of each portion or foods designed particularly for muscles requiring enhanced maximum output values or muscles requiring enhanced motility to target persons.

By comparing differences in masticatory movements between a plurality of foods as described above, it is possible to understand the characteristics of foods and offer foods that meet demands.

<2. Evaluations of Changes in Masticatory Movements Based on Elapse of Chewing Time>

FIG. 14 is a diagram illustrating an example of changes in masticatory movements based on the elapse of chewing time. FIG. 14 illustrates the masticatory movements and their proportions (percentages) based on an elapsed time for a certain food. Note that in FIG. 14, the masticatory movements are selected as those performed with the highest probability on the basis of unit data. Further, "start," "middle," and "end" periods representing the elapsed time are, for example, the three divided periods of the entire time from when chewing of the food starts to the time immediately before swallowing the food. For example, if the entire time is 15 seconds, each of the "start," "middle," and "end" periods is 5 seconds.

According to FIG. 14, it is understood that masticatory movement is most frequently performed in the middle period and is less likely to be performed in the start and end periods during the chewing of the certain food.

Further, according to FIG. 14, a target person who performed the chewing decreased the movement of moving the tongue rightward over time but increased the movement of moving the tongue leftward over time. As a result, it can be estimated that the target person did not continue to chew while keeping the chewing object only on one side of the mouth but performed the masticatory movement while moving the chewing object from side to side during the masticatory movement.

By detecting and evaluating differences in masticatory movements based on an elapsed time as described above, it is possible to grasp the characteristics of masticatory movements by persons. Further, by making comparisons in combination with differences in masticatory movements based on differences in foods, it is also possible to detect comprehensive differences based on the differences in the foods and differences based on an elapsed time (for example, tongue movements remain more active for the food A compared to the food B even when time elapses), and detect the relationships between the characteristics of the foods and the masticatory movements of target persons.

<Display of Masticatory Movements>

FIG. 15 is a diagram illustrating an example of real-time display of masticatory movement analysis. Information in FIG. 15 is shown on, for example, the display 150 of the masticatory movement analysis device 100.

In the real-time display, for example, it is possible to analyze myoelectric potential data while measuring the same during masticatory movements and show the results. The real-time display shows, for example, data file names or other information (such as data owners and types of foods being chewed).

Further, the real-time display shows, for example, a graph G1 illustrating a processing time (an elapsed time since the start of chewing), the number of chewing times (chewing times), and an average pitch or variation (such as distribution and standard deviation).

Further, the real-time display shows the proportion of the 15 masticatory movements performed. As masticatory movement performed, the masticatory movement performed with the highest probability at that timing is determined. Further, the proportion is illustrated in graphs G2 and G3. The graph G2 illustrates, for example, each of the 15 masticatory movements. The graph G3 illustrates the proportion according to the major categories.

Further, the real-time display shows, for example, the history of tongue movements. The history is illustrated in a schematic diagram G4 of the mouth. The history shows, for example, deeper colors when the tongue is present for longer times.

Further, the real-time display shows the muscle workload of each channel (measurement spot). The muscle workload is standardized by being divided by, for example, the amplitude or the like of myoelectric potential data during the exertion of maximum muscle strength, and shows the extent of muscle strength exerted during measurement, or the like. The muscle workload is illustrated in, for example, a graph G5. The graph G5 illustrates, for example, the muscle workload for each measurement spot over time.

Further, the real-time display shows eating-style diagnosis. The eating-style diagnosis comprehensively determines, for example, the tendencies of chewing style (the number of chewing times, chewing frequencies, chewing rhythms, chewing strength, or the like) and the tendencies of eating style (proportions of tongue, cheek, and occlusal movements, unbalanced moving style) and categorizes the same into types. For example, the proportions of masticatory movements for each of the major categories and the elapse of time are illustrated in a graph G6, along with results obtained according to the types.

Note that display contents by the real-time display may be shown after the acquisition of measurement data instead of being shown in real time.

Further, in order to perform the real-time display, a determination processing speed for masticatory movements may be increased. For example, in order to speed up the feature amount calculation processing, calculated feature amounts may be appropriately selected to reduce the number of calculation processes. Further, the effective digit number of measurement data of myoelectric potential may be reduced to simplify a calculation process during feature amount calculation.

[Second Embodiment]

A second embodiment will be described. In the second embodiment, "swallowing" is added as one of determined masticatory movements. "Swallowing" refers to, for example, the movement of swallowing chewed foods. Further, "swallowing" also includes the movement of swallowing not only chewed foods themselves but also water released from the chewed foods, mixtures of the chewed foods and saliva, or the like.

Further, "swallowing" is classified under, for example, "other movements," which is a major category in the classification illustrated in FIG. 10. Further, "swallowing" may also be classified under "throat movements," which is a new major category indicating the movement of moving the throat in the classification illustrated in FIG. 10.

FIG. 16 is a diagram illustrating an example of masticatory movements including swallowing based on differences in chewing objects. Note that FIG. 16 is a diagram illustrating an example where swallowing is added to FIG. 13. In FIG. 16, areas different from those illustrated in FIG. 13 are shaded, and numeric values in FIG. 13 are indicated in brackets.

By adding "swallowing" to the determination of the masticatory movements, the values of "wiping the right teeth (outer side) with the tongue" and "wiping the left teeth (outer side) with the tongue" decreased for both the foods A and B. This is because the movement of "swallowing" is assumed to be similar to the movement of "wiping the right teeth (outer side) with the tongue" and the movement of "wiping the left teeth (outer side) with the tongue."

When "swallowing" is not determined, it is determined as "wiping the right teeth (outer side) with the tongue" and "wiping the left teeth (outer side) with the tongue." "Swallowing" refers to the movement of swallowing objects and does not actually correspond to "wiping the right teeth (outer side) with the tongue" and "wiping the left teeth (outer side) with the tongue." Therefore, by determining "swallowing," the accuracy of determination of masticatory movements increases, enabling movements during "swallowing" to be removed as noise data. This produces the same effects as those obtained when determining "being at rest."

Further, the value of "wiping the molars with both cheeks" decreased for the food A but remained the same for the food B. This is because, for example, it is assumed to be due to the characteristics resulting from the differences between the foods. For example, it is assumed that the food A is a gummy candy and the food B is a gum. The gum itself is not swallowed, but the gummy candy is swallowed. Therefore, when swallowing the gummy candy that is a somewhat large solid object, the movement of swallowing the gum is performed with powerful strength compared to swallowing the gummy candy where only water is swallowed. Therefore, it is assumed that the muscles of both cheeks were needed to move more actively.

Further, in the elapse of a chewing time as well, values change like differences in foods. FIG. 17 is a diagram illustrating an example of changes in masticatory movements including swallowing. Note that FIG. 17 is a diagram illustrating an example where swallowing is added to FIG. 14. In FIG. 17, areas different from those illustrated in FIG. 14 are shaded, and numeric values in FIG. 14 are indicated in brackets. The reason for the changes in the values is the same as that assumed in FIG. 16 described above. Note that in FIG. 17, "swallowing" was 0% in the middle period, showing that "swallowing" was not performed. Therefore, the values of other masticatory movements were not affected.

Note that the data illustrated in FIGS. 16 and 17 is provided as an example only, and movements whose values change (decrease) with the addition of "swallowing" are not limited to "wiping the right teeth (outer side) with the tongue," "wiping the left teeth (outer side) with the tongue," and "wiping the molars with both cheeks" but may include other movements as well. The movements whose values change (decrease) can vary depending on, for example, subjects, target foods, installation conditions of measurement equipment (including the way of placing pads or the like), or the like.

By adding "swallowing" to masticatory movements and classifying the same under "other movements" as described above, the accuracy of the values of the masticatory movements for foods (occlusal movements, tongue movements, and cheek movements) increases, thereby also increasing the accuracy of food evaluation.

Note that training data also needs to include "swallowing" in order to detect "swallowing." For example, a tester causes a subject to intentionally perform masticatory movements including "swallowing," measures myoelectric potential data during swallowing, and performs model learning using the myoelectric potential data itself or feature amounts as training data. By performing model learning using the training data, a learned model can detect "swallowing" movement from the myoelectric potential data.

Further, when movement being performed by a subject is definite during the measurement of myoelectric potential data in an actual operation (for example, in the evaluation of subject's chewing performance) even if the movement is intentionally performed by the subject, the myoelectric potential data in the actual operation may be used as training data.

[Third Embodiment]

A third embodiment will be described. In the third embodiment, measurement spots for myoelectric potential data are changed (added). In the first embodiment, the measurement of myoelectric potential data was targeted at the three spots (the right masseter muscle, the left masseter muscle, and the central portion of the suprahyoid muscle group) of the face. In the third embodiment, the number of measurement spots are increased to enable a further increase in determination accuracy. Note that it is preferable to measure myoelectric potential data from as many measurement spots as possible to increase determination accuracy. However, due to, for example, burdens on a subject, easiness of measurement, accuracy of measurement values, or the like, the myoelectric potential data may not be necessarily measured from all the measurement spots. Therefore, the measurement spots are classified into measurement groups, and will be described below, including measurement priority. Note that in the following description, priority will be expressed as "priority x (where x is an integer)," indicating that the priority is higher as x is smaller and the priority is the same when x is identical.

<1. First Measurement Group>

A first measurement group includes the right masseter muscle, the left masseter muscle, and the central portion of the suprahyoid muscle group. The first measurement group includes the most fundamental measurement spots and is assigned priority 1. For the first measurement group, measurement is easily performed (pads are easily placed), and accuracy of measurement values is high. Further, for the first measurement group, measurement positions are free from interference such as hairs, and reliable measurements are enabled irrespective of age and sex. Moreover, the left and right masseter muscles can also detect the differences between occlusal movements and cheek movements. Further, the central portion of the suprahyoid muscle group can detect the vertical and back-and-forth movements of the tongue or swallowing movements, and also detect the horizontal movements of the tongue.

For example, as illustrated in FIG. 5, the absolute value of the amplitude of the myoelectric potential data from the suprahyoid muscle group becomes extremely smaller compared to the absolute values of the amplitudes of the myoelectric potential data from the left and right masseter muscles. Therefore, for example, if masticatory movements are detected on the basis of only myoelectric potential data from muscles related to the tongue such as the suprahyoid muscle group, the overall accuracy of chewing evaluation may decrease because the accuracy of detecting masticatory movements is low. Therefore, by measuring the myoelectric potential data from the suprahyoid muscle group, which is a muscle related to the tongue, and from the left and right masseter muscles, and by determining masticatory movements as in the first measurement group, the accuracy of detecting chewing movements increases, thereby also increasing the overall accuracy of masticatory evaluation.

<2. Second Measurement Group>

A second measurement group includes the left and right portions of the suprahyoid muscle group (left and right sides of the central portion of the suprahyoid muscle group) and is assigned priority 2. For the second measurement group, measurement is easily performed (pads are easily placed). Further, by measuring the second measurement group, the movements of the tongue can be more accurately detected. Moreover, by measuring the second measurement group, the movements of the tongue associated with generating negative pressure inside the mouth when sucking the cheeks can be detected. As a result, the detection of cheek-sucking movement is enabled.

On the other hand, for example, the movements of the tongue can be measured to some extent at the central portion of the suprahyoid muscle group of the first measurement group. Therefore, the second measurement group is assigned the priority 2.

<3. Third Measurement Group>

A third measurement group includes the left and right temporal muscles (temporal muscles on left and right sides) and is assigned priority 3. By measuring the third measurement group, activity information on various masticatory movements (occlusal movements, tongue movements, and cheek movements) can be supplemented. This is because the lower jaw is stabilized by the action of the temporal muscles, and the tongue can move vertically and horizontally with the lower jaw as a fulcrum. Particularly, by measuring the third measurement group, the movements of the cheeks can be more accurately detected. This is because the movement of wiping the teeth with the cheeks requires the operations of opening the lower jaw and generating negative pressure inside the mouth, both of which are associated with the temporal muscles.

On the other hand, for example, measurement may not be easy such as when pads are difficult to be placed on the temporal muscles due to hairs. Therefore, the third measurement group is assigned the priority 3.

<4. Fourth Measurement Group>

A fourth measurement group includes the left and right cheek muscles (cheek muscles on the left and right side) and is assigned priority 4. By measuring the fourth measurement group, the movement of wiping the teeth with the cheeks can be more accurately detected. Further, by measuring the fourth measurement group, boluses can be retained, or the movements of supporting the horizontal movements of the tongue can be detected.

On the other hand, the cheek muscles may relatively less move compared to the masseter muscles, or interfere with the measurement positions of the masseter muscles, potentially resulting in failure to accurately acquire myoelectric potential. If accurate values cannot be acquired, influence by data loss or errors becomes significant, potentially resulting in a reduction in measurement accuracy. Therefore, the fourth measurement group is assigned the priority 4.

A tester determines measurement spots according to the priorities described above. However, the above priorities are provided as an example only. The measurement spots may be determined depending on, for example, the characteristics (such as age, sex, and conditions or areas of measurement positions) of a subject. Further, the measurement spots may be determined according to masticatory movements needed to be detected.

REFERENCE SIGNS LIST

10: MASTICATORY MOVEMENT ANALYSIS SYSTEM
11: MYOELECTRIC POTENTIAL DATA MEASUREMENT UNIT
12: FEATURE AMOUNT CALCULATION UNIT
13: MASTICATORY MOVEMENT IDENTIFICATION UNIT
14: MODEL LEARNING UNIT
20: MEASUREMENT TARGET PERSON
100: MASTICATORY MOVEMENT ANALYSIS DEVICE
110: CPU
120: STORAGE
121: MYOELECTRIC POTENTIAL MEASUREMENT PROGRAM
122: FEATURE AMOUNT CALCULATION PROGRAM
123: MASTICATORY MOVEMENT IDENTIFICATION MODEL
124: A MODEL LEARNING PROGRAM
130: MEMORY
140: WIRELESS COMMUNICATION CIRCUIT
150: DISPLAY

## Claims

**1.** A chewing movement analysis method comprising:

an acquisition step of measuring myoelectric potential from muscles at a plurality of measurement spots on a face when a target person chews and acquiring myoelectric potential data;
a calculation step of calculating a plurality of feature amounts from unit data obtained by dividing the myoelectric potential data on a basis of a unit time; and
a determination step of determining from the feature amounts which one of tongue movement, cheek movement, and occlusal movement is being performed by the target person during acquisition of the unit data.

**2.** The chewing movement analysis method according to claim 1, wherein the plurality of measurement spots include a right masseter muscle, a left masseter muscle, and a muscle near a central portion of a suprahyoid muscle group.

**3.** The chewing movement analysis method according to claim 1, wherein

the determination step further includes
determining whether a tongue is being moved upward, downward, rightward, or leftward, whether the tongue is being moved forward or backward, and whether the tongue is wiping an inner or outer side of a right or left tooth in the tongue movement, and
determining whether a molar is being wiped by a left cheek, a right cheek, or both the left and right cheeks in the cheek movement.

**4.** The chewing movement analysis method according to claim 1, wherein
the feature amounts are calculated from a waveform of myoelectric potential for each of the measurement spots and from a waveform data group obtained by aggregating myoelectric potential from the plurality of measurement spots.

**5.** The chewing movement analysis method according to claim 1, wherein
first unit data partially overlaps with second unit data, the second unit data being data from a time prior to the first unit data.

**6.** The chewing movement analysis method according to claim 1, wherein
the determination step includes calculating a probability of each movement being performed in the determination as to which one of the movements is being performed.

**7.** The chewing movement analysis method according to claim 1 or 3, wherein
the determination step is performed by a learned model that has performed learning using myoelectric potential data from the plurality of measurement spots obtained when the tongue movement, the cheek movement, and the occlusal movement are performed as training data.

**8.** The chewing movement analysis method according to claim 7, wherein
the training data includes myoelectric potential data from the plurality of measurement spots during a rest time at which chewing is not performed.

**9.** The chewing movement analysis method according to claim 8, wherein
the training data includes myoelectric potential data from the plurality of measurement spots during a swallowing time at which swallowing movement is performed.

**10.** The chewing movement analysis method according to claim 9, wherein
the determination step includes determining that movement during the rest time and movement during the swallowing time do not correspond to any of the tongue movement, the cheek movement, and the occlusal movement.

**11.** The chewing movement analysis method according to claim 2, wherein
the plurality of measurement spots further include left and right sides of the suprahyoid muscle group.

**12.** The chewing movement analysis method according to claim 11, wherein
the plurality of measurement spots further include left and right temporal muscles.

**13.** The chewing movement analysis method according to claim 12, wherein
the plurality of measurement spots further include left and right cheek muscles.

**14.** The chewing movement analysis method according to claim 1, further comprising:
a food difference evaluation step of detecting a difference in chewing movement resulting from a different food type, on a basis of a determination result obtained when the target person chews a different food.

**15.** The chewing movement analysis method according to claim 1, further comprising:
an elapsed time evaluation step of detecting a change in chewing movement over time.

Fig. 1

MASTICATORY MOVEMENT ANALYSIS SYSTEM 10

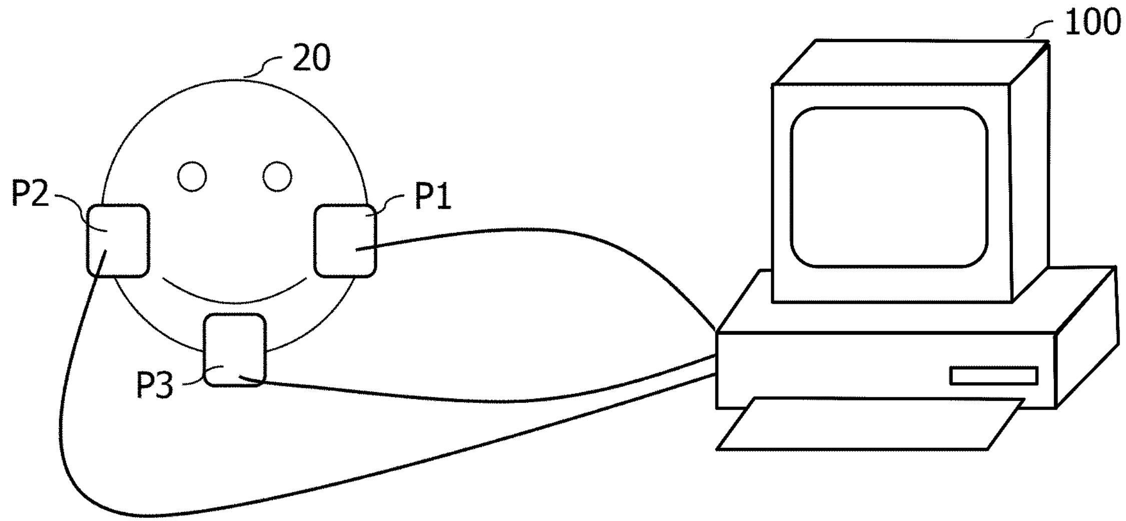

Fig. 2

MASTICATORY MOVEMENT ANALYSIS DEVICE 100

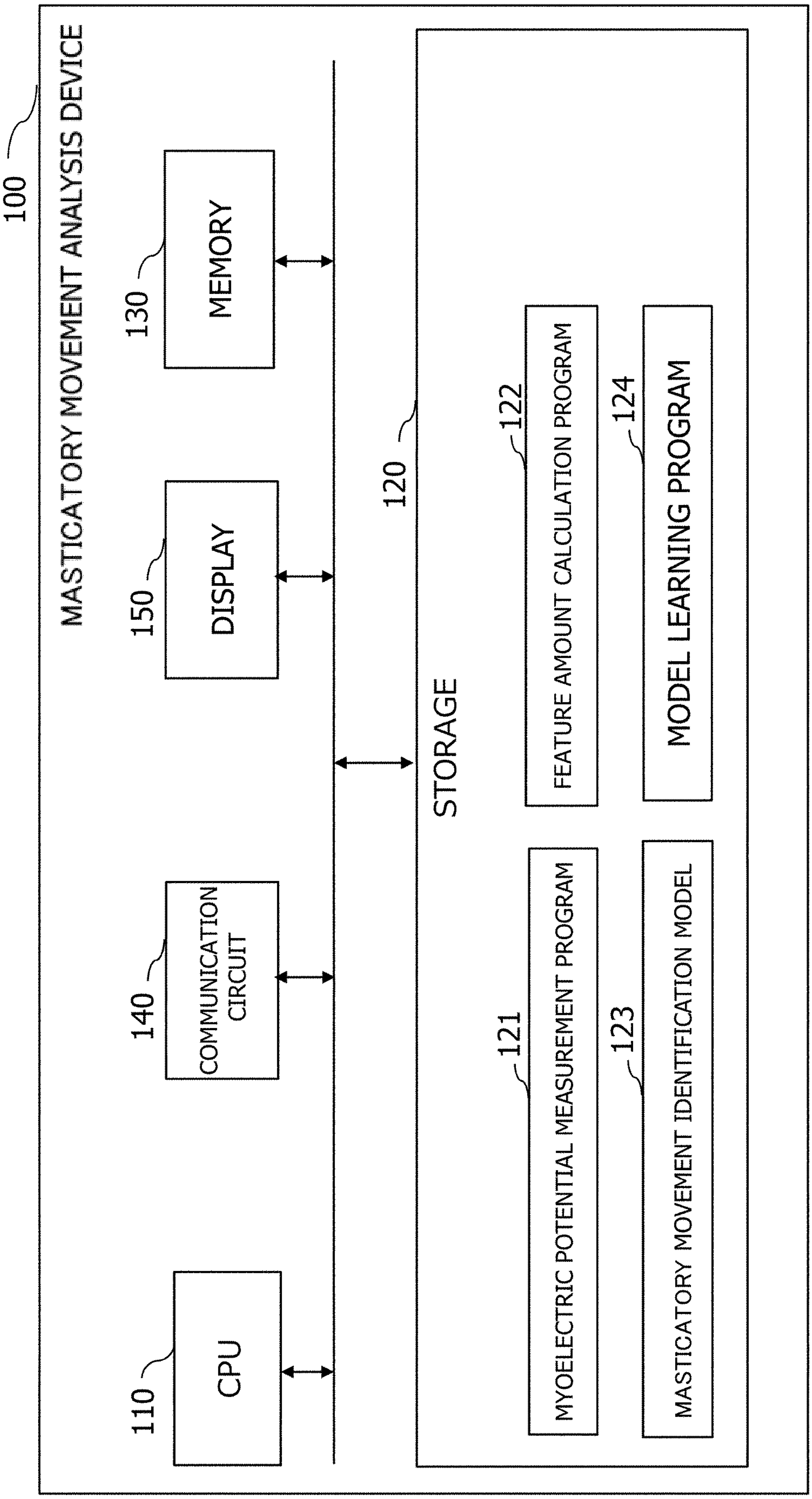

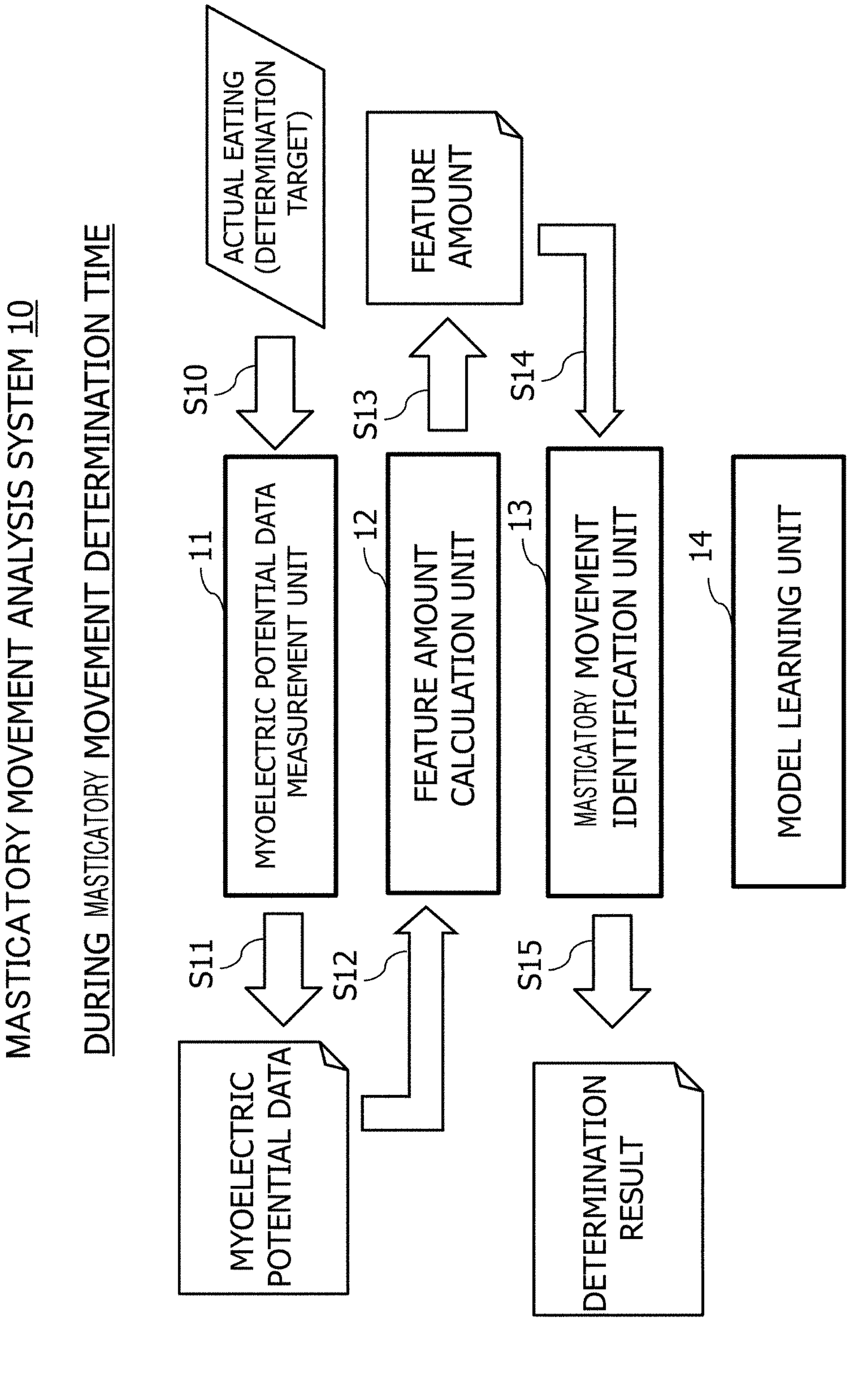
MASTICATORY MOVEMENT ANALYSIS SYSTEM 10
DURING MASTICATORY MOVEMENT DETERMINATION TIME
ACTUAL EATING (DETERMINATION TARGET)
S10
11
MYOELECTRIC POTENTIAL DATA MEASUREMENT UNIT
S11
MYOELECTRIC POTENTIAL DATA
S12
12
FEATURE AMOUNT CALCULATION UNIT
S13
FEATURE AMOUNT
S14
13
MASTICATORY MOVEMENT IDENTIFICATION UNIT
S15
DETERMINATION RESULT
14
MODEL LEARNING UNIT

Fig. 3

Fig. 4

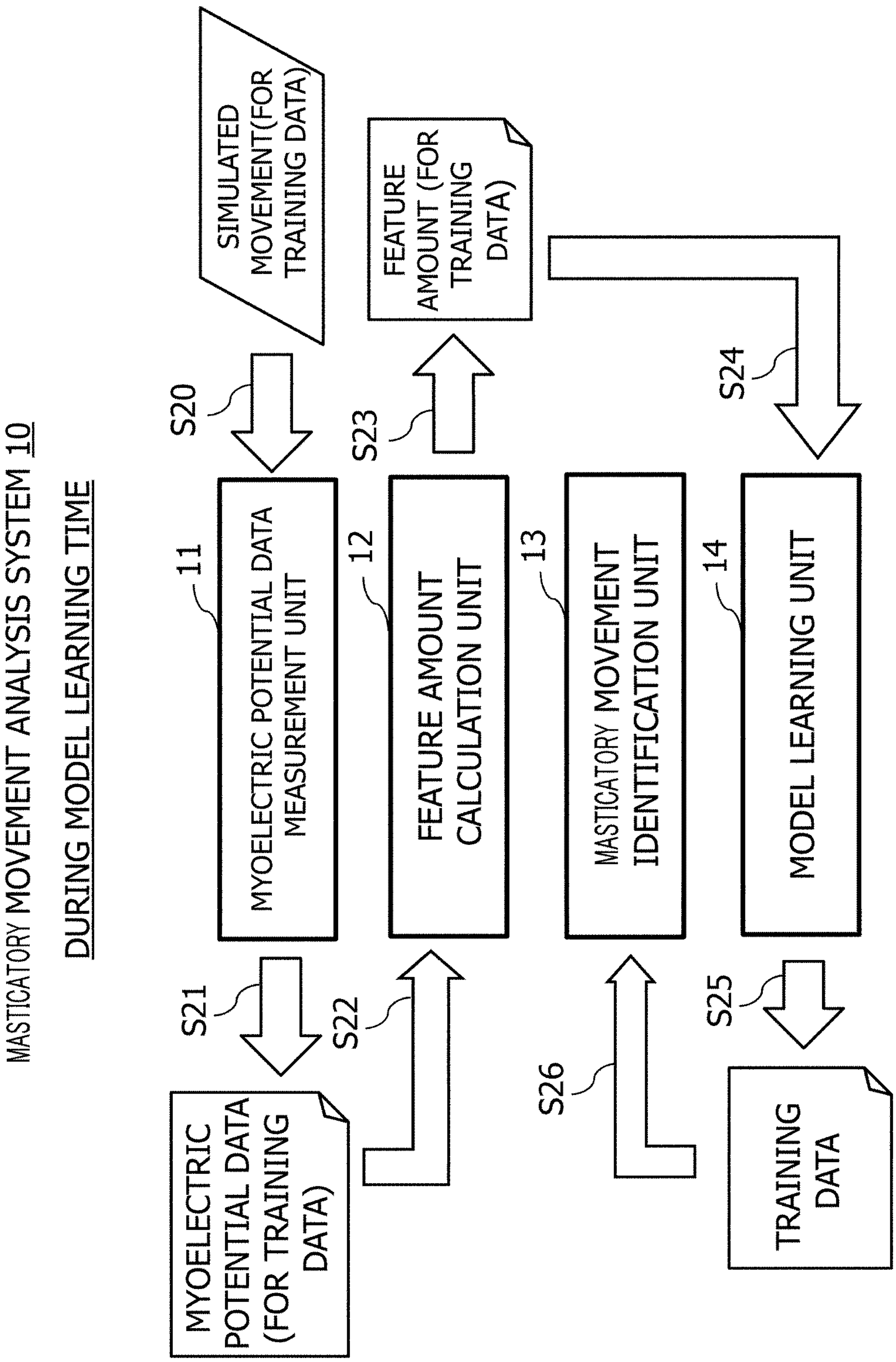
MASTICATORY MOVEMENT ANALYSIS SYSTEM 10
DURING MODEL LEARNING TIME
SIMULATED MOVEMENT(FOR TRAINING DATA)
S20
11
MYOELECTRIC POTENTIAL DATA MEASUREMENT UNIT
S21
MYOELECTRIC POTENTIAL DATA (FOR TRAINING DATA)
S22
12
FEATURE AMOUNT CALCULATION UNIT
S23
FEATURE AMOUNT (FOR TRAINING DATA)
13
MASTICATORY MOVEMENT IDENTIFICATION UNIT
S26
S24
14
MODEL LEARNING UNIT
S25
TRAINING DATA

Fig. 5

MYOELECTRIC POTENTIAL DATA

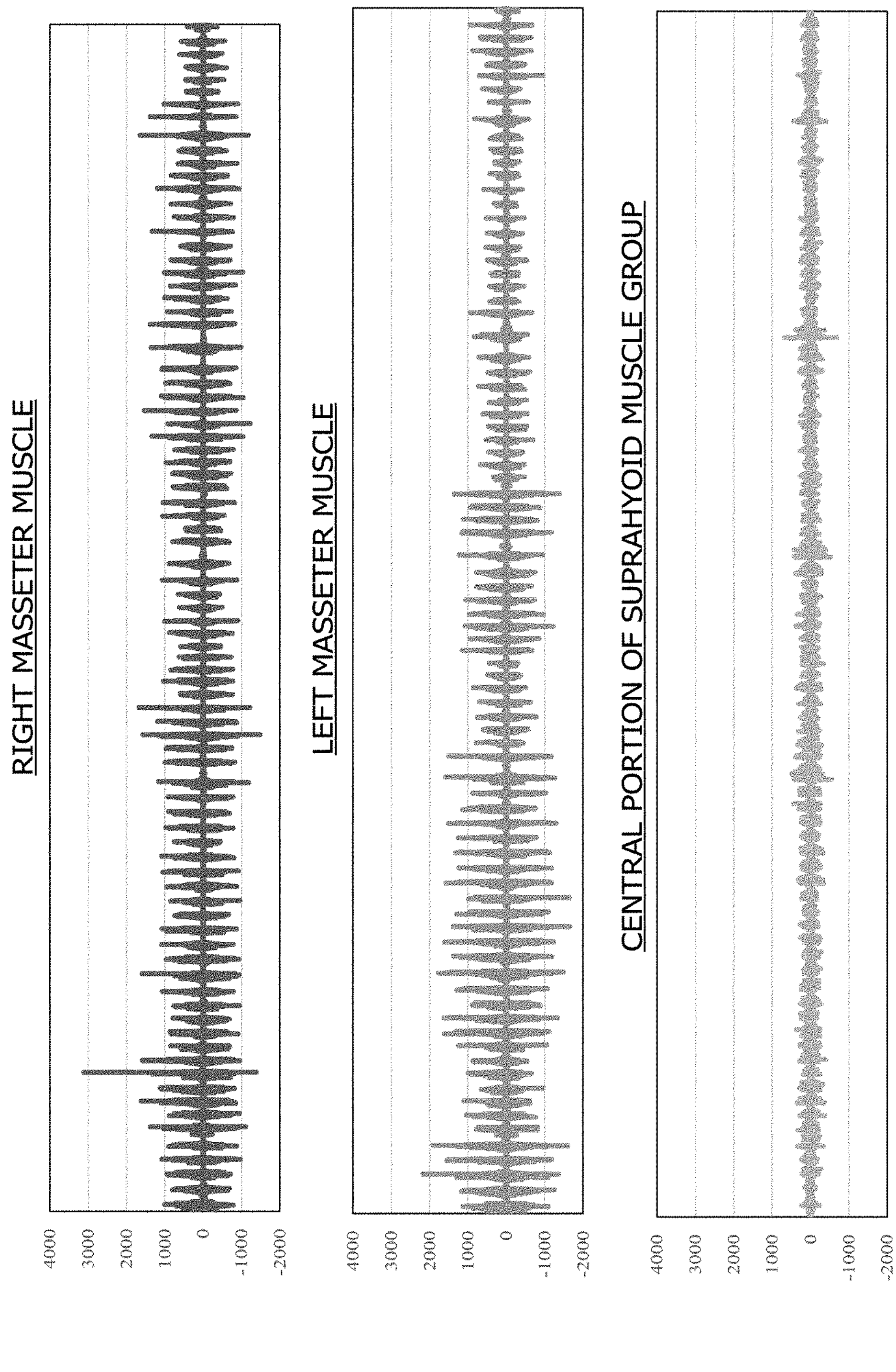

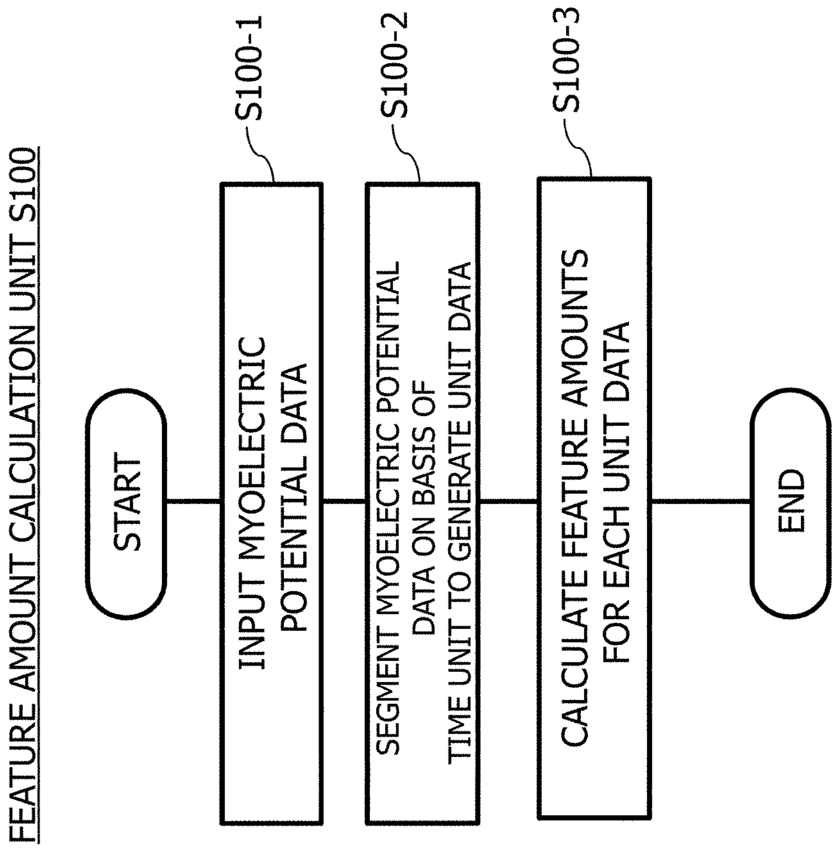
FEATURE AMOUNT CALCULATION UNIT S100
START
INPUT MYOELECTRIC POTENTIAL DATA
S100-1
SEGMENT MYOELECTRIC POTENTIAL DATA ON BASIS OF TIME UNIT TO GENERATE UNIT DATA
S100-2
CALCULATE FEATURE AMOUNTS FOR EACH UNIT DATA
S100-3
END

Fig. 6

Fig. 7

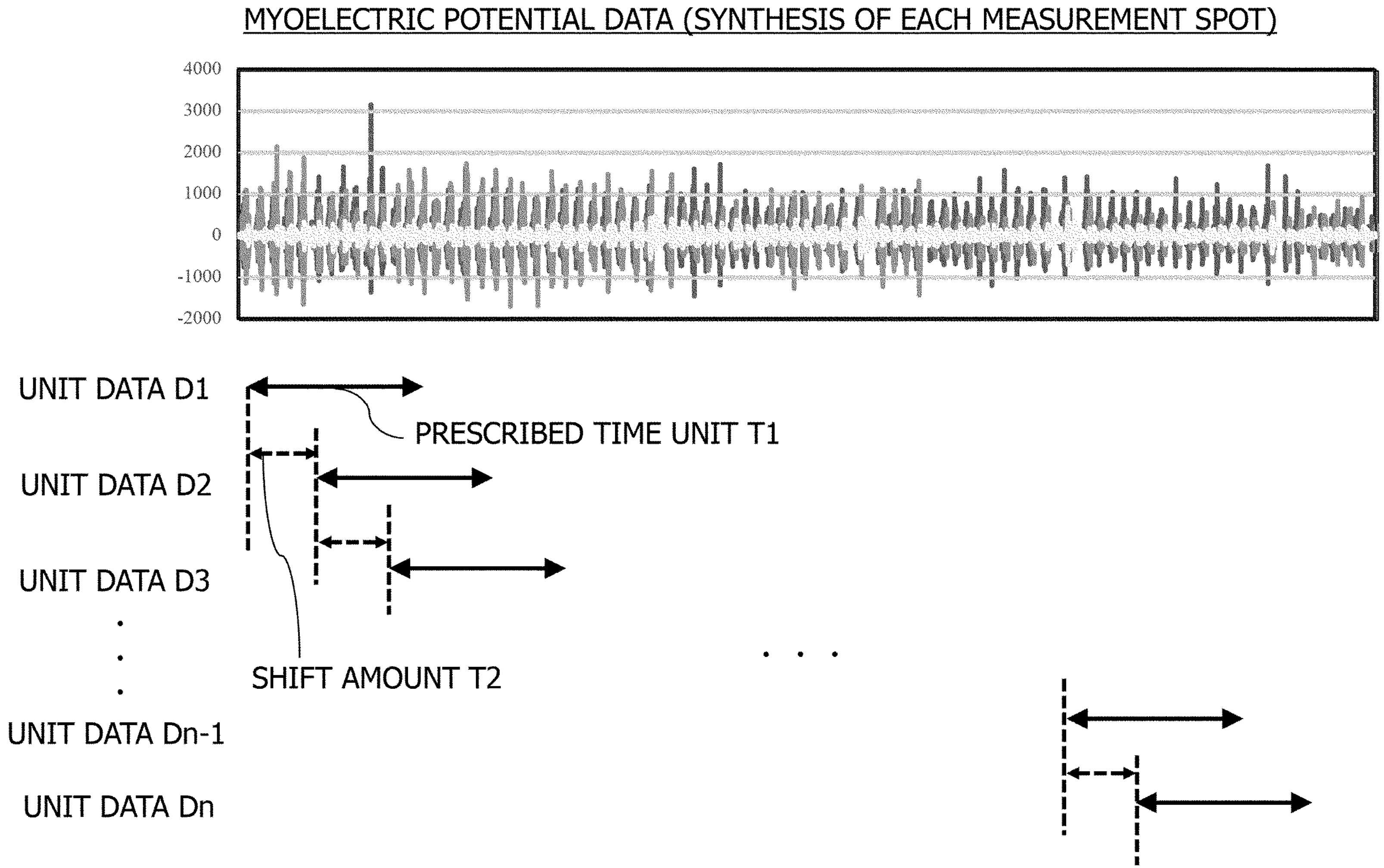
MYOELECTRIC POTENTIAL DATA (SYNTHESIS OF EACH MEASUREMENT SPOT)
4000
3000
2000
1000
0
-1000
-2000
UNIT DATA D1
PRESCRIBED TIME UNIT T1
UNIT DATA D2
UNIT DATA D3
SHIFT AMOUNT T2
UNIT DATA Dn-1
UNIT DATA Dn

Fig. 8

FEATURE AMOUNTS

| **FEATURE AMOUNTS** | **RIGHT MASSETER MUSCLE** | **LEFT MASSETER MUSCLE** | **CENTRAL PORTION OF SUPRAHYOID MUSCLE GROUP** |
|---|---|---|---|
| ROOT MEAN SQUARE | 77.7040 | 85.5562 | 93.2053 |
| MEAN ABSOLUTE VALUE | 45.2044 | 50.7583 | 70.8677 |
| ZERO CROSSING RATE | 403 | 360 | 390 |
| THE NUMBER OF EXTREME VALUES | 935 | 869 | 798 |
| WAVELENGTH | 4.3083e+04 | 4.8782e+04 | 6.8587e+04 |
| FIRST-ORDER COEFFICIENT OF AUTOREGRESSION MODEL | -1.9186 | 1.4828 | -0.6905 |
| SECOND-ORDER | 0.3071 | -0.0957 | 0.0223 |
| THIRD-ORDER | -2.0269 | 1.7196 | -0.8277 |
| FOURTH-ORDER | 0.3258 | -0.1110 | 0.0304 |
| FIFTH-ORDER | -1.9068 | 1.5344 | -0.7368 |
| SIXTH-ORDER | 0.3018 | -0.0999 | 0.0247 |

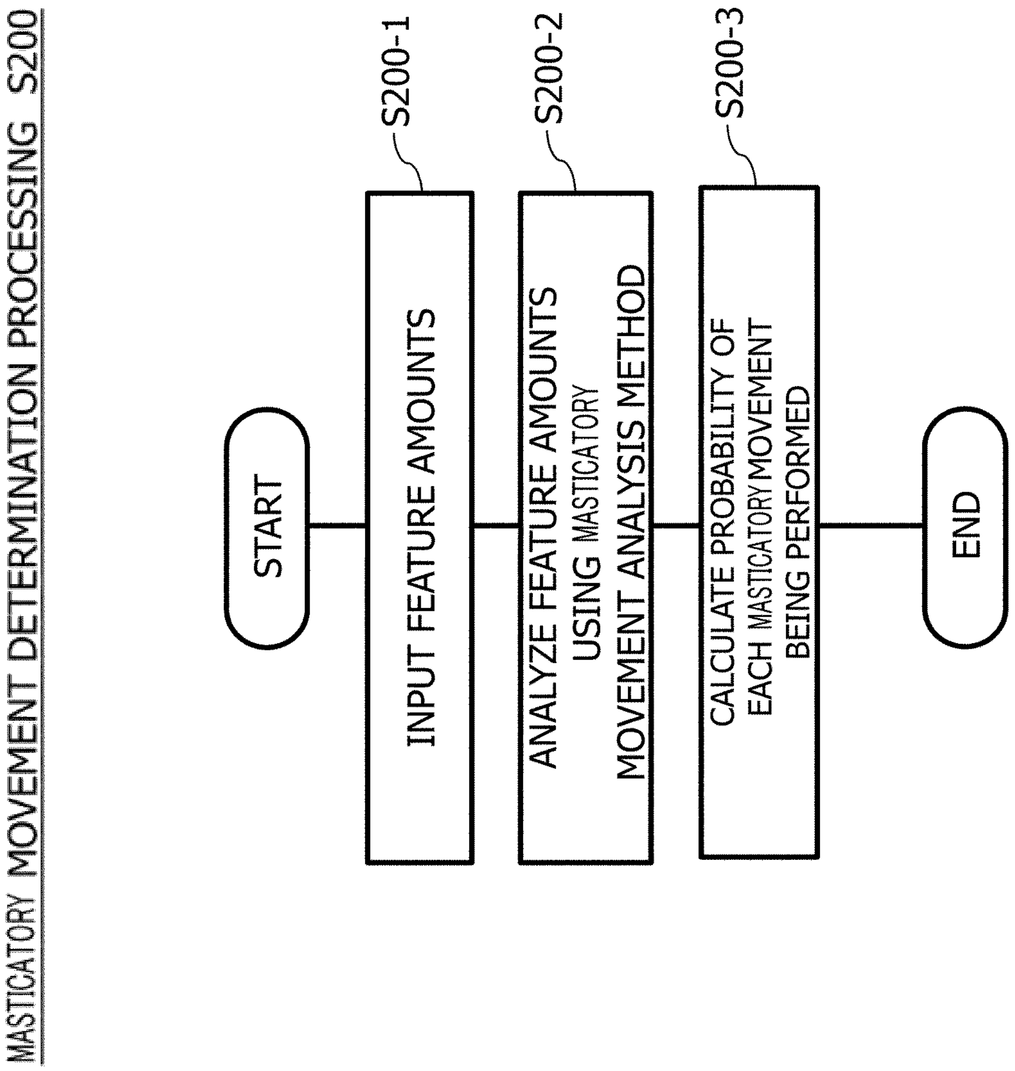
MASTICATORY MOVEMENT DETERMINATION PROCESSING S200
START
INPUT FEATURE AMOUNTS
S200-1
ANALYZE FEATURE AMOUNTS USING MASTICATORY MOVEMENT ANALYSIS METHOD
S200-2
CALCULATE PROBABILITY OF EACH MASTICATORY MOVEMENT BEING PERFORMED
S200-3
END

Fig. 9

Fig. 10

MASTICATORY MOVEMENTS AS DETERMINATION TARGETS

| MAJOR CATEGORIES | DETAILS |
|---|---|
| OCCLUSAL MOVEMENTS | 1.CHEWING |
| CHEEK MOVEMENTS | 2.WIPING MOLARS WITH BOTH CHEEKS (SUCKING BOTH CHEEKS)<br>3.WIPING MOLARS WITH RIGHT CHEEK (SUCKING RIGHT CHEEK)<br>4.WIPING MOLARS WITH LEFT CHEEK (SUCKING LEFT CHEEK) |
| TONGUE MOVEMENTS | 5.UPWARD 6.DOWNWARD 7.FORWARD 8.BACKWARD 9.RIGHTWARD<br>10.LEFTWARD<br>11.WIPING RIGHT TEETH (INNER SIDE OR OCCLUSAL SURFACE)<br>12.WIPING RIGHT TEETH (OUTER SIDE)<br>13.WIPING LEFT TEETH (INNER SIDE OR OCCLUSAL SURFACE)<br>14.WIPING LEFT TEETH (OUTER SIDE) |
| OTHER MOVEMENTS | 15.BEING AT REST |

FIG. 11A

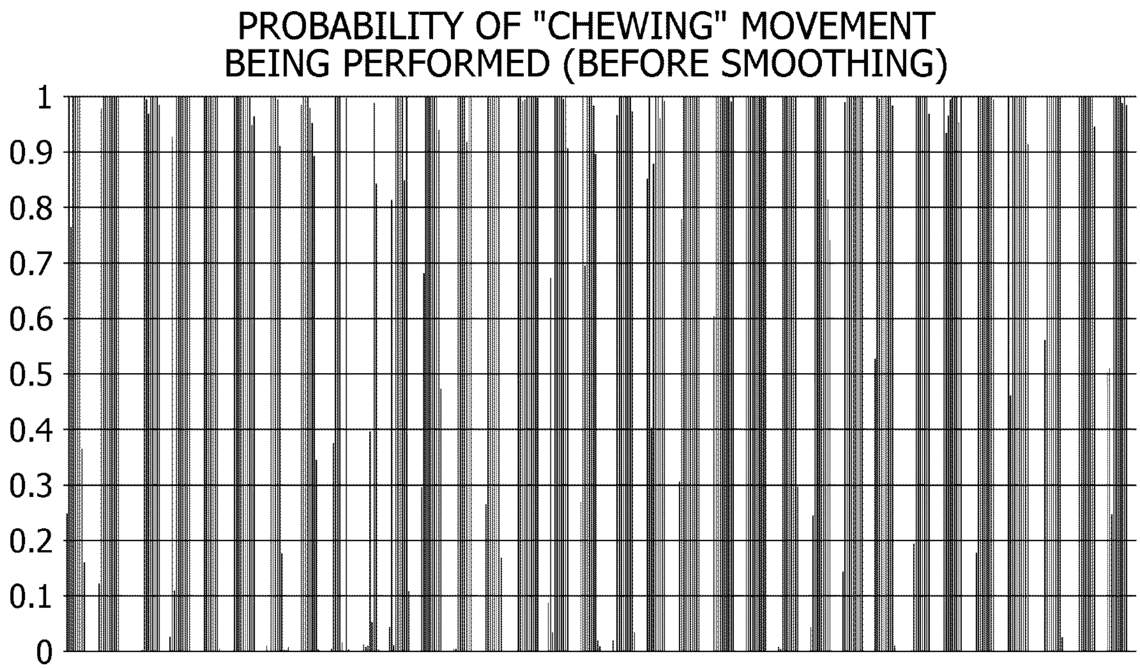


FIG. 11B

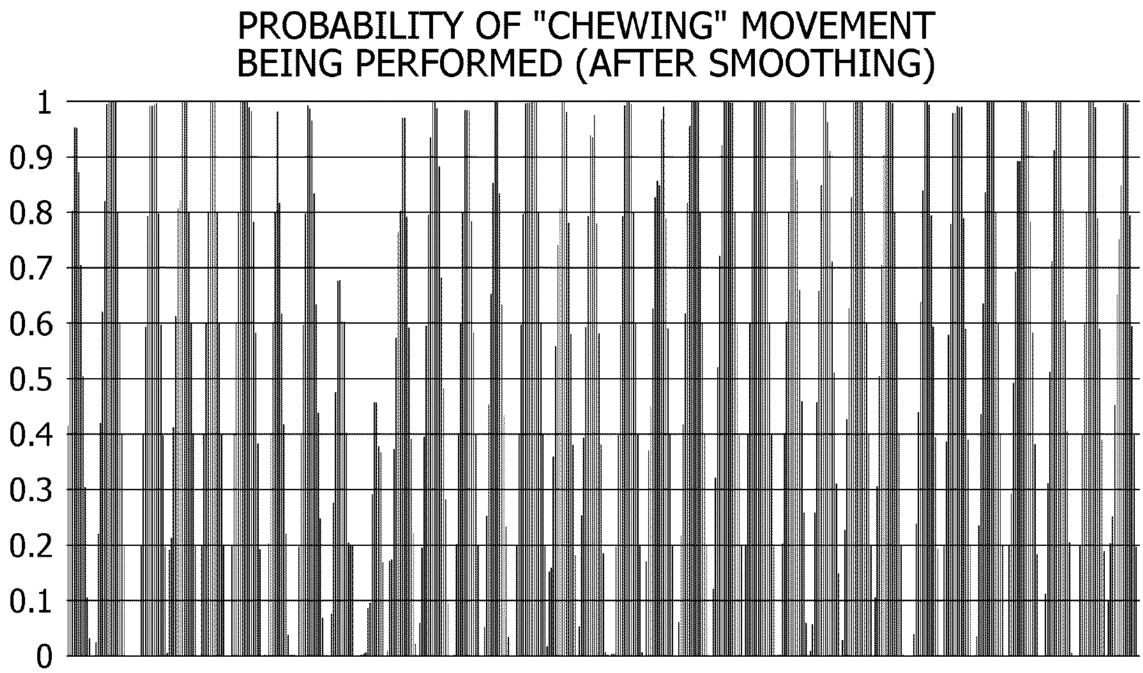

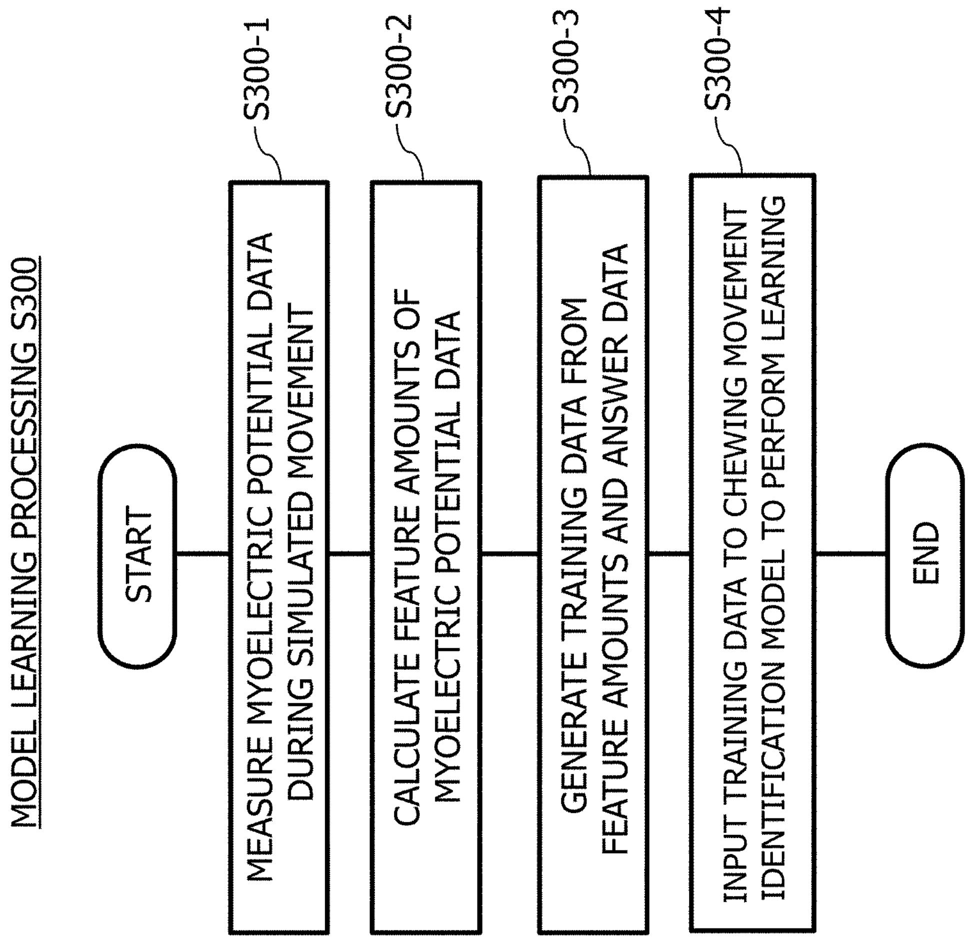
MODEL LEARNING PROCESSING S300
START
MEASURE MYOELECTRIC POTENTIAL DATA DURING SIMULATED MOVEMENT
S300-1
CALCULATE FEATURE AMOUNTS OF MYOELECTRIC POTENTIAL DATA
S300-2
GENERATE TRAINING DATA FROM FEATURE AMOUNTS AND ANSWER DATA
S300-3
INPUT TRAINING DATA TO CHEWING MOVEMENT IDENTIFICATION MODEL TO PERFORM LEARNING
S300-4
END

Fig. 12

Fig. 13

| MOVEMENTS | **DISTRIBUTION** (%) | |
|---|---|---|
| | FOOD A | FOOD B |
| CHEWING | 46.4 | 51.6 |
| WIPING MOLARS WITH BOTH CHEEKS | 2.4 | 4.9 |
| WIPING MOLARS WITH RIGHT CHEEK | 1.8 | 2.4 |
| WIPING MOLARS WITH LEFT CHEEK | 0.0 | 0.1 |
| MOVING TONGUE UPWARD | 4.9 | 7.0 |
| MOVING TONGUE DOWNWARD | 4.5 | 3.1 |
| MOVING TONGUE FORWARD | 1.0 | 0.6 |
| MOVING TONGUE BACKWARD | 2.4 | 2.6 |
| MOVING TONGUE RIGHTWARD | 0.0 | 0.0 |
| MOVING TONGUE LEFTWARD | 2.8 | 1.3 |
| WIPING RIGHT TEETH (INNER SIDE OR OCCLUSAL SURFACE) WITH TONGUE | 0.0 | 0.0 |
| WIPING RIGHT TEETH (OUTER SIDE) WITH TONGUE | 11.7 | 6.4 |
| WIPING LEFT TEETH (INNER SIDE OR OCCLUSAL SURFACE) WITH TONGUE | 0.2 | 0.6 |
| WIPING LEFT TEETH (OUTER SIDE) WITH TONGUE | 21.9 | 19.2 |
| BEING AT REST | 0.0 | 0.1 |

Fig. 14

| MOVEMENTS | **DISTRIBUTION** (%) | | |
| --- | --- | --- | --- |
| | START | MIDDLE | END |
| CHEWING | 49.6 | 54.9 | 50.3 |
| WIPING MOLARS WITH BOTH CHEEKS | 2.5 | 6.3 | 6.0 |
| WIPING MOLARS WITH RIGHT CHEEK | 1.6 | 3.8 | 1.6 |
| WIPING MOLARS WITH LEFT CHEEK | 0.3 | 0.0 | 0.0 |
| MOVING TONGUE UPWARD | 11.2 | 3.3 | 6.6 |
| MOVING TONGUE DOWNWARD | 3.0 | 2.4 | 3.8 |
| MOVING TONGUE FORWARD | 1.1 | 0.8 | 0.0 |
| MOVING TONGUE BACKWARD | 3.3 | 2.2 | 2.5 |
| MOVING TONGUE RIGHTWARD | 0.0 | 0.0 | 0.0 |
| MOVING TONGUE LEFTWARD | 0.8 | 1.6 | 1.4 |
| WIPING RIGHT TEETH (INNER SIDE OR OCCLUSAL SURFACE) WITH TONGUE | 0.0 | 0.0 | 0.0 |
| WIPING RIGHT TEETH (OUTER SIDE) WITH TONGUE | 10.1 | 6.0 | 3.0 |
| WIPING LEFT TEETH (INNER SIDE OR OCCLUSAL SURFACE) WITH TONGUE | 0.8 | 0.0 | 1.1 |
| WIPING LEFT TEETH (OUTER SIDE) WITH TONGUE | 15.6 | 18.8 | 23.5 |
| BEING AT REST | 0.0 | 0.0 | 0.3 |

Fig. 15

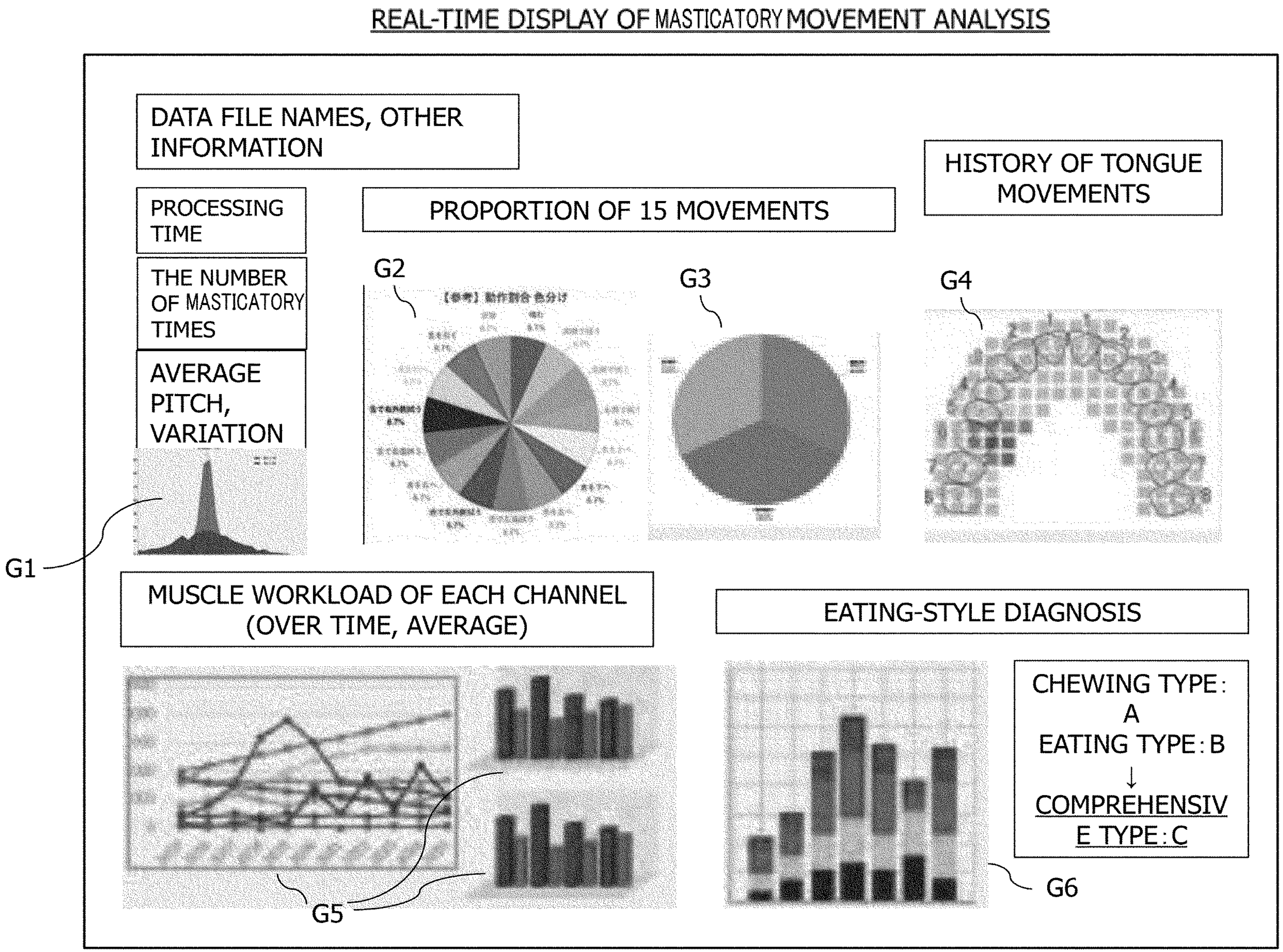
REAL-TIME DISPLAY OF MASTICATORY MOVEMENT ANALYSIS
DATA FILE NAMES, OTHER INFORMATION
PROCESSING TIME
THE NUMBER OF MASTICATORY TIMES
AVERAGE PITCH, VARIATION
G1
PROPORTION OF 15 MOVEMENTS
G2
G3
HISTORY OF TONGUE MOVEMENTS
G4
MUSCLE WORKLOAD OF EACH CHANNEL (OVER TIME, AVERAGE)
G5
EATING-STYLE DIAGNOSIS
CHEWING TYPE : A
EATING TYPE : B
↓
COMPREHENSIVE TYPE : C
G6

Fig. 16

| MOVEMENTS | DISTRIBUTION (%) | |
|---|---|---|
| | FOOD A | FOOD B |
| CHEWING | 46.4 | 51.6 |
| WIPING MOLARS WITH BOTH CHEEKS | 2.2 (2.4) | 4.9 |
| WIPING MOLARS WITH RIGHT CHEEK | 1.8 | 2.4 |
| WIPING MOLARS WITH LEFT CHEEK | 0.0 | 0.1 |
| MOVING TONGUE UPWARD | 4.9 | 7.0 |
| MOVING TONGUE DOWNWARD | 4.5 | 3.1 |
| MOVING TONGUE FORWARD | 1.0 | 0.6 |
| MOVING TONGUE BACKWARD | 2.4 | 2.6 |
| MOVING TONGUE RIGHTWARD | 0.0 | 0.0 |
| MOVING TONGUE LEFTWARD | 2.8 | 1.3 |
| WIPING RIGHT TEETH (INNER SIDE OR OCCLUSAL SURFACE) WITH TONGUE | 0.0 | 0.0 |
| WIPING RIGHT TEETH (OUTER SIDE) WITH TONGUE | 11.3 (11.7) | 6.1 (6.4) |
| WIPING LEFT TEETH (INNER SIDE OR OCCLUSAL SURFACE) WITH TONGUE | 0.2 | 0.6 |
| WIPING LEFT TEETH (OUTER SIDE) WITH TONGUE | 21.5 (21.9) | 17.7 (19.2) |
| BEING AT REST | 0.0 | 0.1 |
| SWALLOWING | 1.4 | 1.8 |

Fig. 17

| MOVEMENTS | DISTRIBUTION(%) | | |
|---|---|---|---|
| | START | MIDDLE | END |
| CHEWING | 49.6 | 54.9 | 50.3 |
| WIPING MOLARS WITH BOTH CHEEKS | 2.5 | 6.3 | 6.0 |
| WIPING MOLARS WITH RIGHT CHEEK | 1.6 | 3.8 | 1.6 |
| WIPING MOLARS WITH LEFT CHEEK | 0.3 | 0.0 | 0.0 |
| MOVING TONGUE UPWARD | 11.2 | 3.3 | 6.6 |
| MOVING TONGUE DOWNWARD | 3.0 | 2.4 | 3.8 |
| MOVING TONGUE FORWARD | 1.1 | 0.8 | 0.0 |
| MOVING TONGUE BACKWARD | 3.3 | 2.2 | 2.5 |
| MOVING TONGUE RIGHTWARD | 0.0 | 0.0 | 0.0 |
| MOVING TONGUE LEFTWARD | 0.8 | 1.6 | 1.4 |
| WIPING RIGHT TEETH (INNER SIDE OR OCCLUSAL SURFACE) WITH TONGUE | 0.0 | 0.0 | 0.0 |
| WIPING RIGHT TEETH (OUTER SIDE) WITH TONGUE | 9.6 (10.1) | 6.0 | 2.7 (3.0) |
| WIPING LEFT TEETH (INNER SIDE OR OCCLUSAL SURFACE) WITH TONGUE | 0.8 | 0.0 | 1.1 |
| WIPING LEFT TEETH (OUTER SIDE) WITH TONGUE | 13.4 (15.6) | 18.8 | 21.0 (23.5) |
| BEING AT REST | 0.0 | 0.0 | 0.3 |
| SWALLOWING | 2.7 | 0.0 | 2.7 |

**INTERNATIONAL SEARCH REPORT**

International application No.
**PCT/JP2023/039485**

**A. CLASSIFICATION OF SUBJECT MATTER**

***A61B 5/11***(2006.01)i
FI: A61B5/11 320

According to International Patent Classification (IPC) or to both national classification and IPC

**B. FIELDS SEARCHED**

Minimum documentation searched (classification system followed by classification symbols)

A61B5/11

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2019/022259 A1 (MELTIN MMI CO., LTD.) 31 January 2019 (2019-01-31) | 1-15 |
| A | JP 2018-871 A (UNIV IWATE) 11 January 2018 (2018-01-11) | 1-15 |
| A | JP 2012-232065 A (UNIV IWATE) 29 November 2012 (2012-11-29) | 1-15 |
| A | JP 2020-148692 A (SNOW BRAND MILK PROD CO LTD) 17 September 2020 (2020-09-17) | 1-15 |
| A | WO 2019/225243 A1 (PANASONIC INTELLECTUAL PROPERTY MANAGEMENT CO., LTD.) 28 November 2019 (2019-11-28) | 1-15 |
| A | CN 111709282 A (COFCO NUTRITION & HEALTH RES INST CO LTD) 25 September 2020 (2020-09-25) | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 December 2023** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/039485**

| Patent document cited in search report | Publication date (day/month/year) | Patent family member(s) | Publication date (day/month/year) |
|---|---|---|---|
| WO 2019/022259 A1 | 31 January 2019 | US 2020/0046284 A1<br>EP 3711667 A1<br>CN 110536636 A | |
| JP 2018-871 A | 11 January 2018 | (Family: none) | |
| JP 2012-232065 A | 29 November 2012 | (Family: none) | |
| JP 2020-148692 A | 17 September 2020 | (Family: none) | |
| WO 2019/225243 A1 | 28 November 2019 | (Family: none) | |
| CN 111709282 A | 25 September 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012232065 A
- WO 2019022259 A